# EUROPÄISCHE PATENTSCHRIFT

(11) **EP 1 658 055 B1**
(45) Veröffentlichungstag und Bekanntmachung des Hinweises auf die Patenterteilung: **14.03.2007**
(21) Anmeldenummer: 04763834.1
(22) Anmeldetag: 05.08.2004
(51) Int. Cl.: A61K 9/20, A61K 31/485, A61K 31/515, A61K 31/5513

(54) **GEGEN MISSBRAUCH GESICHERTE DARREICHUNGSFORM**
FORM OF ADMINISTRATION SECURED AGAINST MISUSE
FORME GALENIQUE EMPECHANT UN USAGE DETOURNE

(30) Priorität: 06.08.2003 DE 10336400
(43) Veröffentlichungstag der Anmeldung: 24.05.2006
(60) Teilanmeldung: 06024704.6
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE); ARKENAU-MARIC, Elisabeth, Dr., 50931 Köln (DE)
(74) Vertreter: Kutzenberger, Helga
(86) Internationale Anmeldenummer: PCT/EP2004/008793
(87) Internationale Veröffentlichungsnummer: WO 2005/016314

(56) Entgegenhaltungen:
- US-A1- 2003 068 392
- US-A1- 2003 124 185
- US-B1- 6 309 668

## Beschreibung

Die vorliegende Erfindung betrifft eine gegen Missbrauch gesicherte, ohne Extrusion thermogeformte Darreichungsform enthaltend neben einem oder mehreren Wirkstoffen mit Missbrauchspotential (A) sowie ggf. physiologisch verträglichen Hilfsstoffen (B) mindestens ein synthetisches oder natürliches Polymer (C) und ggf. mindestens ein Wachs (D), wobei die Komponente (C) sowie die gegebenenfalls vorhandene Komponente (D) jeweils eine Bruchfestigkeit von mindestens 500 N aufweist, sowie ein Verfahren zur Herstellung der erfindungsgemäßen Darreichungsform.

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem Bestimmungszweck entsprechen.
So werden beispielsweise Opiate, die eine exzellente Wirksamkeit bei der Bekämpfung von starken bis sehr starken Schmerzen zeigen, von Mißbrauchern häufig zum Einleiten rauschartiger, euphorisierender Zustände verwendet.

Um Missbrauch zu ermöglichen, werden die entsprechenden Dan-eichungsformen wie Tabletten oder Kapseln vom Missbraucher zerkleinert, z. B. gemörsert, der Wirkstoff aus dem so erhaltenen Pulver mit Hilfe einer vorzugsweise wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Mißbraucher gewünschten Ergebnis, nämlich dem Kick. Dieser Kick wird auch erreicht, wenn die gepulverte Darreichungsform nasal appliziert, d. h. geschnupft wird. Da retardierte, orale Darreichungsformen, die Wirkstoffe mit Mißbrauchspotential enthalten, üblicherweise selbst bei einer oralen Einnahme von missbräuchlich hohen Mengen nicht zu dem vom Mißbraucher gewünschten Kick führen, werden auch diese zum Missbrauch zerkleinert und extrahiert.

Zur Verhinderung des Missbrauchs wurde in dem US-i4- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge an Wirkstoff enthält.

Ein entsprechender Ansatz zur Verhinderung des parenteralen Mißbrauchs liegt auch der in der WO 95/20947 offenbarten Mehrschichttablette zugrunde, die den Wirkstoff mit Mißbrauchspotential und mindestens einen Gelbildner jeweils in unterschiedlichen Schichten getrennt ausweist.

Ein weiterer Ansatz zur Verhinderung des parenteralen Mißbrauchs wird in der WO 031015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll den Mißbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltexon im Fall von Opioiden, oder Verbindungen, die zu physiologischen Abwehrreaktionen führen, wie z. B. Radix Ipecacuanha = Brechwurz, der Darreichungsform zuzusetzen.

Da aber nach wie vor in den meisten Fällen für den Missbrauch, eine Pulverisierung der Darreichungsforinen mit einem zum Missbrauch geeigneten Wirkstoff notwendig ist, war es Aufgabe der vorliegenden Erfindung, die dem Missbrauch vorangehende Pulverisierung der Darreichungsform mit den einem potentiellen Missbraucher üblicherweise zur Verfügung stehenden Mitteln zu erschweren bzw. zu verhindern und somit eine feste Darreichungsform für Wirkstoffe mit Missbrauchspotential zur Verfügung zu stellen, die bei bestimmungsgemäßer Applikation die gewünschte therapeutische Wirkung gewährleistet, aus der aber die Wirkstoffe nicht durch einfaches Pulverisieren in eine zum Missbrauch geeignete Form übergeführt werden können.

Diese Aufgabe wurde durch die Bereitstellung der erfindungsgemäßen, gegen Missbrauch gesicherten, ohne Extrusion thermogeformten Darreichungsform, die neben einem oder mehreren Wirkstoffen mit Mißbrauchspotential (A) mindestens ein synthetisches oder natürliches Polymer (C) und ggf. mindestens ein Wachs (D) enthält, wobei die Komponente (C) und die gegebenenfalls vorhandene Komponente (D) jeweils eine Bruchfestigkeit von mindestens 500 N aufweist, gelöst.

Durch den Einsatz von Polymeren mit der angegebenen Mindestbruchfestigkeit (gemessen, wie in der Anmeldung angegeben), vorzugsweise in solchen Mengen, dass auch die Darreichungsform eine solche Mindestbruchfestigkeit von mindestens 500 N aufweist, gelingt es, ein Pulverisieren der Darreichungsform mit üblichen Mitteln zu verhindern und damit den anschließenden Missbrauch erheblich zu erschweren bzw. zu unterbinden.

Ohne ausreichende Zerkleinerung ist nämlich eine parenteral, insbesondere intravenöse, gefahrlose Applikation nicht möglich oder die Extraktion des Wirkstoffes daraus dauert für den Missbraucher zu lange bzw. ein Kick bei missbräuchlicher, oralen Einnahme erfolgt nicht, da keine spontane Freisetzung passiert.

Unter einer Zerkleinerung wird erfindungsgemäß die Pulverisierung der Darreichungsform mit üblichen Mitteln, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung verstanden.

Die erfindungsgemäße Darreichungsform ist daher zur Verhinderung des parenteralen, nasalen und/oder oralen Missbrauchs von Wirkstoffen, vorzugsweise von pharmazeutischen Wirkstoffen, mit Mißbrauchspotential geeignet.

Pharmazeutische Wirkstoffe mit Mißbrauchspotential sind dem Fachmann ebenso wie deren einzusetzende Mengen und Verfahren zu deren Herstellung bekannt und können als solche, in Form ihrer dementsprechenden Derivate, insbesondere Ester oder Ether, oder jeweils in Form entsprechender physiologisch verträglicher Verbindungen, insbesondere in Form ihrer entsprechenden Salze oder Solvate, als Racemate oder Stereoisomere in der erfindungsgemäßen Darreichungsform vorliegen. Die erfindungsgemäße Darreichungsform eignet sich auch für die Verabreichung von mehreren pharmazeutischen Wirkstoffen in einer Darreichungsform. Vorzugsweise erhält die Darreichungsform nur einen bestimmten Wirkstoff.

Die erfindungsgemäße Darreichungsform eignet sich insbesondere zur Verhinderung des Missbrauchs wenigstens eines pharmazeutischen Wirkstoffs, der ausgewählt ist aus der Gruppe umfassend Opioide, Tranquillantien, vorzugsweise Benzodiazepine, Barbiturate, Stimulantien und weitere Betäubungsmittel.

Ganz besonders eignet sich die erfindungsgemäße Darreichungsform zur Verhinderung des Mißbrauchs eines Opioids, Tranquillanz oder eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-l-methyl-6-phenyl-4H [1,2,4]triazolo[4,3-a][1,4]-benzodiazepin (Alprazolam), 2-Diethylaminopropiophenon (Amfepramon), (±)-a-Methylphenethylamin (Amfetamin), 2-(a-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5a-epopxy-7a[(S)-1-hydroxy-1,2,2-trimethyl-propyl]-6-methoxy-6,14-*endo*-ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1 S,2S)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1 -methyl-1 H-thieno[2,3-e][1,4]diazepin-2(3*H*)-on (Clotiazepam), 10-Chlor-11b-(2chlorphenyl)-2,3,7,11b-tetrahydrooxazolo[3,2-d][1,4]benzodiazepin-6(5*H*)-on (Cloxazolam), (-)-Methyl-[3β-benzoyloxy-2β(1aH,5a*H*)-tropancarboxylat] (Cocain), 4,5a-Epoxy-3-methoxy-1 7-methyl-7-morphinen-6a-ol (Codein), 5-(1-Cyclohexenyl)-5-ethylbarbitursäure (Cyclobarbital),* Cyclorphan, Cyprenorphin, 7-Chlor 5-(2-chlorphenyl)-1H 1,4-benzodiazepin-2(3I)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5a-Epoxy 3-methoxy-17-methyl-6a-morphinanol (Dihydrocodein), 4,5a-Epoxy-17-methyl-3,6a-morphinandiol (Dihydromorphin), Dimenoxadol, Dimepheptanol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6a-ol (Ethylmorphin), Etonitazen, 4,5a Epoxy-7a-(1-hydroxy-1-methylbutyl)-6-methoxy-17-methyl-6,14-*endo*-etheno-morphinan-3-ol (Etorphin), *N-*Ethyl-3-phenyl-8,9,10-trinorboman-2-ylamin(Fencamfamin),7-[2-(α-Methylphenethylamino)ethy]-theophyllin) (Fenet-yllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phehyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*, 6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1 (4*H*)-on (Loprazolam), 7-Chlor-5-(2-chlorphenyl)-3-hydroxy-1*H* 1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3H-imidazo[2,1-a]isoindol-5-oi (Mazindol), 7-Chlor 2,3-dihydro-1-methyl-5-phenyl-1 H 1,4-benzodiazepin (Medazepam), N-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,a-Dimethylphenethylamin (Metamfetamin), (±)-6-Dimethylamino-4,4-diphenyl-3-heptanon (Methadon), 2-Methyl-3-o-tolyi-4(3H)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [b, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis*-*trans*)-10-Chlor-2,3,7,11b-tetrahydro-2-methyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pemolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyt)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), a,a-Dimethylphenethylamin (Phentermin), 7-Chlor-5-phenyl-1-(2-propinyl)-1*H*-1,4-benzodiazepin-2(3H)-on (Pinazepam), a-(2-Piperidyl)benzhydrylalkohol (Pipradrol), 1'-(3-Cyan-3,3₋dtphenylpropyl)[1,4'-bipiperidin]-4'-carboxamid (Piritramid), 7-Chlor-1-(cyclopropylmethyl)-5-phenyl-1H-1,4-benzodiazepin-2(3H)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(N-phenylpropanamido)piperidino]propanoat} (Remifentanil), 5-sec-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor 2-hydroxy-methyl-5-phenyl-1*H*-1,4-benzodiazepin-2(3*H*)-on (Temazepam), 7-Chlor 5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4H-[1,2,4]triazolo[4,3-a][1,4]benzodiazepin (Triazolam), 5-(1-Methylbutyl)-5-vinylbarbitursäure (Vinylbital), (1 R,2R)-3-(3-Dimethylamino-1 -ethyl-2-methyl-propyl)-phenol, (1 R, 2R, 4S)-2-[Dimethylamino)methyl-4-(p-fluorbenzyloxy)-1-(m-methoxyphenyl)cyclohexanol, (1R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-D)methylamnomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-d)methy)aminomethyl-1-hydroxy-cyctohexyl-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxycyclohexyl)-phenyl ester, (RR-SS)-2',4'-Difluoro-3-hydroxy biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäße Darreichungsform eignet sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride.

Weiterhin eignet sich die erfindungsgemäße Darreichungsform insbesondere zur Verhinderung des Missbrauchs eines opioiden Wirkstoffes ausgewählt aus der Gruppe umfassend (1R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1 RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

Zur Erzielung der notwendigen Bruchfestigkeit der erfindungsgemäßen Darreichungsform werden mindestens ein synthetisches oder natürliches Polymer (C) mit einer Bruchfestigkeit, gemessen nach der in der vorliegenden Anmeldung offenbarten Methode, von mindestens 500 N eingesetzt. Bevorzugt wird hierfür mindestens ein Polymeres ausgewählt aus der Gruppe umfassend Polyalkylenoxide, vorzugsweise Polymethylenoxid, Polyethylenoxid, Polypropylenoxid; Polyethylen, Polypropylen, Polyvinylchlorid, Polycarbonat, Polystyrol, Polyacrylat, deren Copolymerisate und Mischungen aus mindestens zwei der genannten Polymeren eingesetzt. Bevorzugt sind hochmolekulare, thermoplastische Polyalkylenoxide. Besonders bevorzugt sind hochmolekulare Polyethylenoxide mit einem Molekulargewicht von mindestens 0,5 Mio., vorzugsweise mindestens 1 Mio. bis 15 Mio., bestimmt durch rheologische Messungen. Diese Polymeren weisen eine Viskosität bei 25 °C von 4500 bis 17600 cP, gemessen an einer 5 Gew.% wässrigen Lösung mit Hilfe eines Brookfield Viskosimeter, Model RVF (Spindel Nr. 2 / Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 / Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden bevorzugt als Pulver eingesetzt. Sie können in Wasser löslich sein.

Des weiteren können zusätzlich zur Erzielung der notwendigen Bruchfestigkeit der erfindungsgemäßen Darreichungsform mindestens ein natürliches oder synthetisches Wachs (D) mit einer Bruchfestigkeit, gemessen nach der in der vorliegenden Anmeldung offenbarten Methode, von mindestens 500 N eingesetzt werden. Bevorzugt sind die Wachse mit einem Erweichungspunkt von mindestens 60°C. Besonders bevorzugt sind Camaubawachs und Bienenwachs. Ganz besonders bevorzugt ist Camaubawachs. Camaubawachs ist ein natürliches Wachs, das aus den Blättern der Camaubapalme gewonnen wird und einen Erweichungspunkt von wenigstens = 80°C aufweist. Beim zusätzlichen Einsatz der Wachskomponente wird diese zusammen mit wenigstens einem Polymeren (C) in solchen Mengen eingesetzt, dass die Darreichungsform eine Bruchfestigkeit von mindestens 500 N aufweist.

Vorzugsweise wird die Komponente (C) in einer Menge von 35 bis 99,9 Gew.%, besonders bevorzugt von wenigstens 50 Gew.%, ganz besonders bevorzugt von wenigstens 60 Gew.%, bezogen auf das Gesamtgewicht der Darreichungsform, eingesetzt.

Als Hilfsstoffe (B) können die üblichen für die Formulierung von festen Darreichungsformen bekannten Hilfsstoffe verwendet werden. Vorzugsweise sind dies Weichmacher, wie Polyethylenglykol, Hilfsstoffe, die Wirkstofffreisetzung beeinflussend, vorzugsweise hydrophobe oder hydrophile, vorzugsweise hydrophile Polymere, ganz besonders bevorzugt Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Die erfindungsgemäßen Darreichungsformen zeichnen sich dadurch aus, dass sie aufgrund ihrer Härte mit Hilfe von üblichen, einem Missbraucher zur Verfügung stehenden Zerkleinerungsmitteln, wie Mörser und Pistill, nicht zu pulverisieren sind. , Ein oraler, parenteraler, inbesondere intravenöser oder nasaler Missbrauch ist dadurch praktisch ausgeschlossen. Um jedoch jeden möglichen Missbrauch der erfindungsgemäßen Darreichungsformen vorzubeugen, können die erfindungsgemäßen Darreichungsformen in einer bevorzugten Ausführungsform als Hilfsstoffe (B) weitere Missbrauchs-erschwerende bzw. -verhindernde Mittel enthalten.

So kann die erfindungsgemäße, gegen Missbrauch gesicherte Darreichungsform, die neben einem oder mehreren Wirkstoffen mit Missbrauchspotential, mindestens einem härtebildenden Polymer (C) und ggf. mindestens einen Wachs (D) noch wenigstens eine der nachfolgenden Komponenten (a)-(e) als Hilfsstoffe (B) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum.
(e) wenigstens einen Farbstoff als aversives Mittel
(f) wenigstens einen Bitterstoff

Die Komponenten (a) bis (f) sind jeweils für sich allein zusätzlich zur Sicherung der erfindungsgemäßen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen, Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten, gelingt es, bei erfindungsgemäßen Darreichungsformen noch effektiver den Missbrauch zu erschweren.

In einer Ausführungsform kann die erfindungsgemäße Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die erfindungsgemäße Darreichungsform sämtliche Komponenten (a)-(f) aufweisen.

Sofern die erfindungsgemäße Darreichungsform gegen Missbrauch die Komponente (a) umfasst, kommen als den Nasen- und/oder Rachenraum reizende Stoffe erfindungsgemäß sämtliche Stoffe in Betracht, die bei entsprechender Applikation über den Nasen- und/oder Rachenraum eine Reaktion des Körpers hervorrufen, die entweder für den Missbraucher so unangenehm ist, dass er die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so daß der Mißbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben. Die entsprechende Beschreibung wird hiermit als Referenz eingeführt und gilt als Teil der Offenbarung.

Unter Darreichungseinheit wird eine separate bzw. separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonytsenföl, und von diesen Inhaltsstoffen abgeleiteten Verbindungen.

Vorzugsweise kann die erfindungsgemäße Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.
Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.-%, bezogen auf das Gesamtgewicht der Darreichungseinheit.

Eine weitere Möglichkeit bei der erfindungsgemäßen Darreichungsform gegen Missbrauch vorzubeugen, besteht darin, wenigstens ein viskositätserhöhendes Mittel als weitere Missbrauchs-verhindemde Komponente (b) der Darreichungsform zuzusetzen, das in einem mit Hilfe einer notwendigen Mindestmenge an einer wässrigen Flüssigkeit, vorzugsweise als ein aus der Darreichungsform gewonnenes wässriges Extrakt, ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, Wirkstoff-haltige Gel beim Einbringen vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wäßriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, daß eine parenterale, insbesondere intravenöse, Applikation gefahrlos möglich ist. In Kombination mit mindestens einer ggf. vorhandenen Komponente (a) bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schäden des Missbrauchers führen.

Zur Überprüfung, ob ein viskositätserhöhendes Mittel als Komponente (b) zur Anwendung in der erfindungsgemäßen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur zusätzlichen Missbrauchs-Vorbeugung bzw. - Verhinderung bei den erfindungsgemäßen Darreichungsformen.

Sofern der erfindungsgemäßen Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalline Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkemmehl (Cesagum^{®} LA-200, Cesagum^{®} LID/150, Cesagum^{®} LN-1), Pektine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401)®), Guarkemmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150^{®}), Tarakemmehl (Polygum 43/1^{®}), Propylenglykolalginat (Protanal-Ester SD-LB^{®}),
Natrium-Hyaluronat, Tragant, Tacagummi (Vidogum SP 200^{®}), fermentiertes Polysaccharid- Welan Gum (K1A96), Xanthan-Gummi (Xantural 180^{®}). Xanthane sind besonders bevorzugt. Die in Klammem angegebenen Bezeichnungen sind die Handelsnamen; unter denen die jeweiligen Materialien am Markt geführt sind. Im allgemeinen ist eine Menge von 0,1 bis 20 Gew.%, besonders bevorzugt 0,1 bis 15 Gew.%, bezogen auf das Gesamtgewicht der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von ≥ 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wässriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge einer wässrigen Flüssigkeit, ein Gel bildet.

Es ist auch möglich, die viskositätserhöhenden Mittel und die übrigen Bestandteile in räumlich voneinander getrennter Anordnung in der erfindungsgemäßen Darreichungsform zu formulieren.

Des weiteren kann die erfindungsgemäße Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäßen Darreichungsform vorliegen und keine Wirkung bei bestimmungsgemäßer Verwendung entfalten.

Geeignete Antagonisten zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von ≥ 1 mg, besonders bevorzugt in einer Menge von 3 bis 100 mg, ganz besonders bevorzugt in einer Menge von 5 bis 50 mg auf pro Darreichungsform, d.h. pro Dosiereinheit eingesetzt.

Weist die erfindungsgemäße Darreichungsform als Wirkstoff ein Stimulanz auf, ist der Antagonist bevorzugt ein Neuroleptikum, vorzugsweise wenigstens eine Verbindung ausgewählt aus der Gruppe bestehend aus Haloperidol, Promethacin, Fluophenazin, Perphenazin, Levomepromazin, Thioridazin, Perazin, Chlorpromazin, Chlorprothixin, Zuclopentixol, Flupentixol, Prothipendyl, Zotepin, Benperidol, Pipamperon, Melperon und Bromperidol.

Vorzugsweise weist die erfindungsgemäße Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur Vorbeugung und Sicherung der erfindungsgemäßen Darreichungsform gegen Mißbrauch die Komponente (d) umfaßt, kann sie wenigstens ein Emetikum aufweisen, das vorzugsweise in einer räumlich getrennten Anordnung von den übrigen Komponenten der erfindungsgemäßen Darreichungsform vorliegen und bei bestimmungsgemaßer Anwendung keine Wirkung im Körper entfalten sollte.

Geeignete Emetika zur Verhinderung des Missbrauchs eines Wirkstoffs sind dem Fachmann an sich bekannt und können als solche oder in Form entsprechender Derivate, insbesondere Ester oder Ether, oder jeweils in Form entsprechender physiologisch.verträglicher Verbindungen, insbesondere in Form ihrer Salze oder Solvate in der erfindungsgemäßen Danreiehungsform vorliegen.

In der erfindungsgemäßen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffe$ Emetin, in Betracht, wie sie z.B. in "Pharmazeutische Biologie - Drogen und ihre inhaltsstoffe" von Prof. Dr. Hildebert Wagner, 2., bearbeite Auflage, Gustav Fischer Verlag. Stuttgart, New York 1982 beschrieben werden. Die entsprechende Literaturbeschreibung wird hiermit als Referenz eingeführt und gilt als Teil der Offenbarung.

Vorzugsweise kann die erfindungsgemäße Darreichungsform als Komponente (d) das Emetikum Emetin aufweisen, bevorzugt in einer Menge von ≥ 3 mg, besonders bevorzugt ≥ 10 mg und ganz besonders bevorzugt in einer Menge von ≥ 20 mg pro. Darreichungsform, d.h. Dosiereinheit.

Ebenfalls bevorzugt kann als Emetikum Apomorphin in der erfindungsgemäßen Missbrauchssicherung zum Einsatz kommen, vorzugsweise in einer Menge von vorzugsweise 2: 3 mg, besonders bevorzugt > 5 mg und ganz besonders bevorzugt 2: 7 mg pro Dosiereinheit.

Sofern die erfindungsgemäße Darreichungsform die Komponente (e) als weiteren missbrauchsverhindemden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 031015531 zu entnehmen, wobei die entsprechende Offenbarung als Teil der vorliegenden Offenbarung gelten soll und hiermit als Referenz eingeführt wird.

Sofern die erfindungsgemäße Darreichungsform als zusätzlichen Missbrauchsverhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen, deren entsprechende Offenbarung als Offenbarung der vorliegenden Anmeldung gelten soll und hiermit als Referenz eingeführt wird. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex®). Besonders bevorzugt ist Denatonium-Benzoat.

Die erfindungsgemäße feste Darreichungsform eignet sich zur oralen, vaginalen oder rektalen, vorzugsweise zur oralen Einnahme. Vorzugsweise ist sie nicht filmförmig.

Die erfindungsgemäße Darreichungsform kann in multipartikulärer Form, bevorzugt in Form von Mikrotabletten, Mikrokapseln, Mikropellets, Granulaten, Sphäroiden, Perlen oder Pellets, ggf. in Kapseln abgefüllt oder zu Tabletten verpreßt, vorzugsweise zur oralen Verabreichung, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform werden ggf. auch die üblichen Hilfsstoffe (B) zur Formulierung der Darreichungsform mitverwendet.
Die erfindungsgemäß gegen Missbrauch gesicherte, feste Darreichungsform wird vorzugsweise ohne Extruder-Einsatz hergestellt, indem vorzugsweise die Komponenten (A), (B), (C) und die ggf. vorhandene Komponente (D) sowie ggf. mindestens eine der ggf. vorhandenen weiteren missbrauchsverhindemden Komponenten (a) - (f) mitgemischt oder, wenn notwendig, separat unter Zugabe der Komponente (C) und gegebenenfalls der Komponente (D) gemischt werden und die resultierende Mischung bzw. die resultierenden Mischungen ggf. nach einer Granulierung zu der Darreichungsform unter vorangehender oder gleichzeitiger Wärmeeinwirkung durch Krafteinwirkung geformt wird bzw. werden.

Diese Erwärmung und Krafteinwirkung zur Herstellung der Darreichungsform erfolgt ohne Extruder-Einsatz.

Die Mischung der Komponenten (A), (B), (C) und ggf. (D) sowie bzw. der ggf. vorhandenen weiteren Komponenten (a) - (f) und ggf. der Komponenten (C) und der ggf. vorhandenen Komponente (D) erfolgt ggf. jeweils in einem dem Fachmann bekannten Mischgerät. Das Mischgerät kann beispielsweise ein Wälzmischer, Schüttelmischer, Schermischer oder Zwangsmischer sein.

Die resultierende Mischung bzw. die resultierenden Mischungen wird (werden) vorzugsweise direkt zu der erfindungsgemäßen Darreichungsform unter vorangehender oder gleichzeitiger Wärmeeinwirkung durch Krafteinwirkung geformt. Beispielsweise kann die Mischung durch Direkttablettierung zu Tabletten geformt werden. Bei einer Direkttablettierung unter gleichzeitiger Wärmeeinwirkung wird mit Hilfe des Tablettierwerkzeugs, d. h. des Unterstempels, Oberstempels und der Matrize die zu verpressende Mischung zumindest bis zum Erweichungspunkt der Polymeren-Komponente (C) erhitzt und dabei verpresst. Bei einer Direkttablettierung unter vorangehender Wärmeeinwirkung wird das zu verpressende Gut unmittelbar vor der Tablettierung mindest bis zur Erweichungstemperatur der Komponete (C) erhitzt und anschließend mit Hilfe des Tablettierwerkzeugs verpresst.

Die resultierende Mischung aus den Komponenten (A), (B), (C) und ggf. der Komponente (D) sowie der ggf. vorhandenen Komponenten (a) bis (f) bzw. der Mischung mindestens einer dieser Komponenten (a) bis (f) mit der Komponente (C) kann auch zuerst granuliert und anschließend unter vorangehender oder gleichzeitiger Wärmeeinwirkung unter Krafteinwirkung zu der erfindungsgemäßen Darreichungsform geformt werden.

Bei jeder Krafteinwirkung erfolgt diese solange, bis die Darreichungsform eine Bruchhärte von mindestens 500 N erreicht hat.

Die Granulierung kann durch Feuchtgranulation oder Schmelzgranulation in bekannten Granulatoren durchgeführt werden.

Jede der erwähnten Verfahrensschritte, insbesondere die Erwärmungen und gleichzeitige oder nachfolgende Krafteinwirkung zur Herstellung der erfindungsgemäßen Darreichungsform erfolgt ohne Extruder-Einsatz.

In einer weiteren bevorzugten Ausführungsform liegt die erfindungsgemäße Darreichungsform in Form einer Tablette, einer Kapsel oder in Form eines oralen osmotischen therapeutischen Systems (OROS) vor, vorzugsweise wenn mindestens noch eine weitere missbrauchsverhindemde Komponente (a) - (f) vorhanden ist.

Sofern die Komponenten (c) und/oder (d) und/oder (f) in der erfindungsgemäßen Darreichungsform vorhanden sind, ist darauf zu achten, dass sie so formuliert oder so gering dosiert sind, daß sie bei bestimmungsgemäßer Applikation der Darreichungsform praktisch keine den Patienten oder die Wirksamkeit des Wirkstoffs beeinträchtigende Wirkung entfalten können.

Wenn die erfindungsgemäße Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung bei einem Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäßen Darreichungsform zur Sicherung der Darreichungsform der Einsatz der Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen den (die) Wirkstoff(e), mindestens ein Polymer (C) und die gegebenenfalls vorhandene Komponente (D) und gegebenenfalls wenigstens eine der gegebenenfalls vorhandenen Komponenten (a) und/oder (b) und/oder (e) bzw. jeweils wenigstens ein Polymer (C) und gegebenenfalls (D) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass jede der genannten Untereinheiten nach den vorstehend angegebenen Verfahren formuliert werden.

Ein wesentlicher Vorteil der getrennten Formulierung der Wirkstoffe von den Komponenten (c) bzw. (d) bzw. (f) in Untereinheiten (X) und (Y) der erfindungsgemäßen Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C) und gegebenenfalls die Komponente (D) enthalten und in der vorstehend angegebenen Weise formuliert wurden.

Sollte es den Missbrauchem wider Erwarten gelingen, eine solche erfindungsgemäße Darreichungsform, welche die Komponenten (c) und/oder (e) und/oder (d) und/oder (f) in Untereinheiten (Y) aufweist, zum Zwecke der mißbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die Formulierung einer erfindungsgemäßen Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäßen Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) und gegebenenfalls (D) mit formuliert und die Formulierung gemäß den vorstehend angegebenen Verfahren zur Erzielung der notwendigen Härte durchgeführt wird.

In einer bevorzugten Ausführungsform der erfindungsgemäßen Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y), z. B. durch mechanische Auslese, möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multpartikulärer Form können auch bevorzugt in eine Kapsel abgefüllt oder zu einer Tablette verpreßt werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

Die multipartikulären Untereinheiten können auch als Slurry oder als Suspension in pharmazeutisch unbedenklichen Suspensionsmedien als orale Darreichungsform formuliert werden.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäßen Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so daß neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäße Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die der erfindungsgemäßen Härteanforderung genügen muss, in ein- und derselben multipartikulären Form formuliert sind. In einer weiteren bevorzugten Ausführungsform der erfindungsgemäßen Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäße Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäße Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette oder eines Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss die erfindungsgemäßen Härtevoraussetzungen erfüllen.

Ebenfalls besonders bevorzugt ist eine Ausführungsform der erfindungsgemäßen Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt. Die entsprechenden Beschreibungen werden hiermit als Referenz eingeführt und gelten als Teil der Offenbarung.

In einer weiteren bevorzugten Ausführungsform hat die Untereinheit (X) der erfindungsgemäßen Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der Formulierung der erfindungsgemäßen Darreichungsform jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der erfindungsgemäßen Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen sind, dem Fachmann an sich bekannt

Sofern die Freisetzung der Komponente (c) und/oder (d) und/oder (f) aus der . Untereinheit (Y) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und gegebenenfalls (D) zur Erfüllung der Härtebedingung der erfindungsgemäßen Darreichungsform enthält.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.
Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1). Die entsprechenden Beschreibungen werden hiermit als Referenz eingeführt und gelten als Teil der Offenbarung.

Ggf. können die vorstehend genannten Materialien mit weiteren üblichen, dem Fachmann bekannten Hilfsstoffen, vorzugsweise ausgewählt aus der Gruppe umfassend Weichmacher, Gleitmittel, Antioxidantien, wie z. B. Glycerinmonostearat, halbsynthetische Triglyceridderivate, halbsynthetische Glyceride, hydriertes Rizinusöl, Glycerinpalmitostearat, Glycerinbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierte Triglyceride, Glyceride, Polyoxyalkylenglykole, Polyalkylenglykole und deren Derivate abgemischt werden.

Sofern die erfindungsgemäße Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäße Darreichungsform weist eine kontrollierte Freisetzung des Wirkstoffes auf. Sie eignet sich dabei vorzugsweise für eine 2x tägliche Verabreichung an Patienten.

Die erfindungsgemäße Darreichungsform kann einen oder mehrere Wirkstoffe mit Missbrauchspotential zumindest teilweise in einer darüber hinaus retardierten Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, daß die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf. vorhandenen Komponente (c) und/oder (d) eine beeinträchtigende Wirkung entfalten können. Außerdem darf durch die Zugabe von retardierenden Materialien keine Beeinträchtigung der notwendigen Härte erfolgen.

Die kontrollierte Freisetzung aus der erfindungsgemäßen Darreichungsform wird vorzugsweise durch Einbettung des Wirkstoffes in eine Matrix erzielt. Die als Matrixmaterialien dienenden Hilfsstoffe kontrollieren die Wirkstofffreisetzung. Matrixmaterialien können beispielweise hydrophile, gelbildende Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethem oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch die Komponenten (C) und ggf. vorhandene Komponente (D), die zur Erzielung der erfindungsgemäß notwendigen Bruchfestigkeit von mindestens 500 N dienen, bereits als zusätzliche Matrixmaterialien dienen.

Sofern die erfindungsgemäße Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst.
Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt. Die entsprechende Literaturbeschreibung wird hiermit als Referenz eingeführt und gilt als Teil der Offenbarung.

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Materials und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

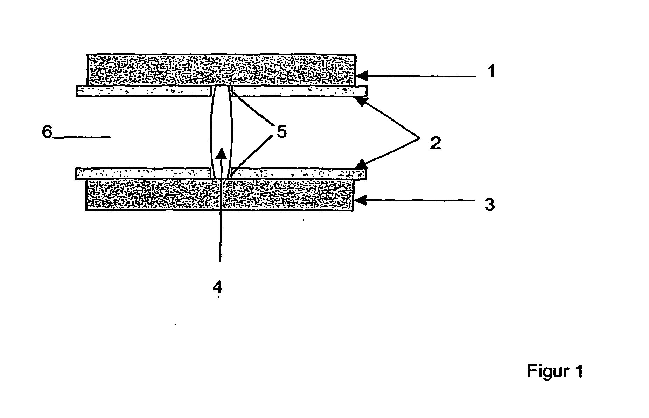

Figur 1 zeigt die Messung der Bruchfestigkeit einer Tablette, insbesondere die dafür eingesetzte Justierungsvotrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar , aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiele:

In einer Reihe von Beispielen wurde Tramadolhydrochlorid als Wirkstoff verwendet. Tramadolhydrochlorid wurde, obwohl Tramadol kein Wirkstoff mit üblichem Mißbrauchspotential ist, da es nicht unter das Betäubungsmittelgesetz fällt, aber wodurch das experimenteller Arbeiten erleichtert wird. Tramadol ist außerdem ein Vertreter der Klasse der Opioide mit ausgezeichneter Wasserlöslichkeit.

### Beispiel 1

| Komponenten | Pro Tablette | Gesamtansatz |
|---|---|---|
| Tramadolhydrochlorid | 100 mg | 100 g |
| Polyethylenoxid, NF, MG 7 000 000 (Polyox WSR 303, Dow Chemicals) | 200 mg | 200 g |
| Gesamtgewicht | 300 mg | 300 g |

Tramadolhydrochlorid und Polyethylenoxidpulver wurden in einem Freifallmischer gemischt. Ein Tablettierwerkzeug mit Oberstempel, Unterstempel und Matrize für Tabletten mit 10 mm Durchmesser und einem Wölbungsradius von 8 mm wurde in einem Heizschrank auf 80°C erhitzt. Mittels des erhitzten Werkzeugs wurden jeweils 300 mg der Pulvermischung verpreßt, wobei der Preßdruck für mindestens 15 s aufrechterhalten wurde durch Einspannen des Tablettierwerkzeugs in einen Schraubstock.

Die Bruchfestigkeit der Tabletten wurde gemäß der angegebenen Methode mit der angegebenen Apparatur bestimmt. Bei einer Krafteinwirkung von 500 N trat kein Bruch der Tabletten auf.
Die Tablette konnte mit einem Hammer nicht zerkleinert werden. Dies war auch mit Hilfe von Mörser und Pistill nicht möglich.

Die in-vitro-Freisetzung des Wirkstoffs aus der Zubereitung wurde in der Blattrührerapparatur nach Pharm. Eur. bestimmt. Die Temperatur des Freisetzungsmediums betrug 37°C und die Umdrehungsgeschwindigkeit des Rührers 75 min ⁻¹. Zu Beginn der Untersuchung wurde jede Tablette in jeweils 600 ml künstlichen Magensaft pH 1,2 gegeben. Nach 30 Minuten wurde durch Zugabe von Lauge der pH-Wert auf 2,3 erhöht, nach weiteren 90 Minuten auf pH 6,5 und nach nochmals 60 weiteren Minuten auf pH 7,2. Die jeweils zu einem Zeitpunkt im Lösungsmedium befindliche freigesetzte Menge des Wirkstoffs wurde spektralphotometrisch bestimmt.

| Zeit | Freigesetzte Menge |
|---|---|
| 30 min | 15 % |
| 240 min | 52 % |
| 480 min | 80 % |
| 720 min | 99 % |

### Beispiel 2

Die Pulvermischung aus Beispiel 1 wurde in Portionen zu 300 mg auf 80°C erhitzt und in die Matrize des Tablettierwerkzeugs eingefüllt. Anschließend erfolgte die Verpressung. Die Tablette weist dieselben Eigenschaften auf wie die Tablette nach Beispiel 1 hergestellt.

### Beispiel 3

| Rohstoff | Pro Tablette | Gesamtansatz |
|---|---|---|
| Tramadolhydrochlorid | 50 mg | 100 g |
| Polyethylenoxid, NF, MG 7 000 000 (Polyox WSR 303, Dow Chemicals) | 100 mg | 200 g |
| Gesamtgewicht | 150 mg | 300 g |

Tramadolhydrochlorid und die vorstehend angegebenen Komponenten wurden in einem Freifallmischer gemischt. Ein Tablettierwerkzeug mit Oberstempel, Unterstempel und Matrize für Tabletten mit 7 mm Durchmesser wurde in einem Heizschrank auf 80°C erhitzt. Mittels des erhitzten Werkzeugs wurden jeweils 150 mg der Pulvermischung verpreßt, wobei der Preßdruck für mindestens 15 s durch Einspannen des Tablettierwerkzeugs in einen Schraubstock aufrechterhalten wurde.

Die Bruchfestigkeit der Tabletten wurde gemäß der angegebenen Methode mit Hilfe der angegebenen Apparatur bestimmt. Bei einer Krafteinwirkung von 500 N trat kein Bruch der Tabletten auf.

Die in-vitro-Freisetzung des Wirkstoffs wurde wie in Beispiel 1 bestimmt und betrug:

| Zeit | Freigesetzte Menge |
|---|---|
| 30 min | 15 % |
| 240 min | 62 % |
| 480 min | 88 % |
| 720 min | 99 % |

### Beispiel 4

| Rohstoff | Pro Tablette | Gesamtansatz |
|---|---|---|
| Tramadolhydrochlorid | 100 mg | 100 g |
| Polyethylenoxid, NF, MG 7 000 000 (Polyox WSR 303, Dow Chemicals) | 180 mg | 180 g |
| Xanthan, NF | 20 mg | 20 g |
| Gesamtgewicht | 300 mg | 300 g |

Tramadolhydrochlorid, Xanthan und Polyethylenoxid wurden in einem Freifallmischer gemischt. Ein Tablettierwerkzeug mit Oberstempel, Unterstempel und Matrize für Tabletten mit 10 mm Durchmesser und einem Wölbungsradius von 8 mm wurde in einem Heizschrank auf 80°C erhitzt. Mittels des erhitzten Werkzeugs wurden jeweils 300 mg der Pulvermischung verpreßt, wobei der Preßdruck für mindestens 15 s durch Einspannen des Tablettierwerkzeugs ein einen Schraubstock aufrechterhalten wurde.

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf. Die Tabletten wurden etwas plastisch verformt.

Die in-vitro-Freisetzung des Wirkstoffs aus der Zubereitung wurde wie in Beispiel 1 bestimmt und betrug:

| Zeit | Freigesetzte Menge |
|---|---|
| 30 min | 14 % |
| 240 min | 54 % |
| 480 min | 81 % |
| 720 min | 99 % |

Die Tabletten konnten mit einem Messer in Stücke mit bis zu ca. 2 mm Kantenlänge zerschnitten werden. Eine weitere Zerkleinerung bis zur Pulverisierung war nicht möglich. Beim Versetzen der Stücke mit Wasser bildet sich ein hochviskoses Gel. Das Gel war nur sehr schwer durch eine Injektionskanüle von 0,9 mm zu pressen. Beim Einspritzen des Gels in Wasser mischte sich das Gel nicht spontan mit Wasser, sondern blieb visuell unterscheidbar.

### Beispiel 5

| Rohstoff | Pro Tablette | Gesamtansatz |
|---|---|---|
| Tramadolhydrochlorid | 50 mg | 100 g |
| Polyethylenoxid, NF, MG 7 000 000 (Polyox WSR 303, Dow Chemicals) | 90 mg | 180 g |
| Xanthan, NF | 10 mg | 20 g |
| Gesamtgewicht | 300 mg | 300 g |

Tramadolhydrochlorid, Xanthan und Polyethylenoxid wurden in einem Freifallmischer gemischt. Ein Tablettierwerkzeug mit Oberstempel, Unterstempel und Matrize für Oblongtabletten mit 10 mm Länge und 5 mm Breite wurde in einem Heizschrank auf 90°C erhitzt. Mittels des erhitzten Werkzeugs wurden jeweils 150 mg der Pulvermischung verpreßt, wobei der Preßdruck für mindestens 15 s durch Einspannen des Tablettierwerkzeugs in einen Schraubstock aufrechterhalten.

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf. Die Tabletten wurden etwas plastisch verformt.

Die in-vitro-Freisetzung des Wirkstoffs aus der Zubereitung wurde wie in Beispiel 1 bestimmt und betrug:

| Zeit | Freigesetzte Menge |
|---|---|
| 30 min | 22 % |
| 120 min | 50 % |
| 240 min | 80 % |
| 360 min | 90 % |
| 480 min | 99 % |

Die Tabletten konnten zu Stückchen bis zu ca. 2 mm Kantenlänge zerschnitten, aber nicht pulverisiert werden. Bei Versetzen der Stücke mit Wasser bildet sich ein hochviskoses Gel. Das Gel war nur sehr schwer durch eine Injektionskanüle von 0,9 mm zu pressen. Bei Einspritzen des Gels in Wasser mischte sich das Gel nicht spontan mit Wasser, sondern blieb visuell unterscheidbar.

### Beispiel 6

Wie in Beispiel 1 beschrieben, wurde eine Tablette mit folgender Zusammensetzung hergestellt:

| Komponenten | ProTablette | Pro Ansatz |
|---|---|---|
| Oxycodon Hydrochlorid | 20,0 mg | 0,240 g |
| Xanthan NF | 20,0 mg | 0,240 g |
| Polyethylenoxid, NF, MG 7 000 000 (Polyox WSR 303 Dow Chemicals) | 110,0 mg | 1,320 g |
| Gesamtgewicht | 150,0 mg | 1,800 g |

Die Freisetzung des Wirkstoffs wurde wie folgt bestimmt:

Die in-vitro-Freisetzung des Wirkstoffs aus der Tablette wurde in der Blattrührerapparatur nach Pharm. Eur. bestimmt. Die Temperatur des Freisetzungsmediums betrug 37°C und die Umdrehungsgeschwindigkeit 75 U pro Minute. Als Freisetzungsmedium diente der in der USP beschriebene Phosphatpuffer pH 6,8. Die zum jeweiligen Prüfzeitpunkt im Lösungsmittel befindliche Menge des Wirkstoffs wurde spektralphotometrisch bestimmt.

| Zeit | Mittelwert |
|---|---|
| 0 min | 0% |
| 30 min | 17 % |
| 240 min | 61 % |
| 480 min | 90 % |
| 720 min | 101,1 % |

Die Bruchfestigkeit der Tabletten wurde gemäß der angegebenen Methode mit Hilfe der angegebenen Apparatur gemessen. Bei einer Krafteinwirkung von 500 N trat kein Bruch der Tabletten auf.

Die Tabletten konnten zu Stückchen bis zu ca. 2 mm Kantenlänge zerschnitten, aber nicht pulverisiert werden. Bei Versetzen der Stücke mit Wasser bildet sich ein hochviskoses Gel. Das Gel war nur sehr schwer durch eine Injektionskanüle von 0,9 mm zu pressen. Bei Einspritzen des Gels in Wasser mischte sich das Gel nicht spontan mit Wasser, sondern blieb visuell unterscheidbar.

### Beispiel 7:

| Komponenten | Pro Tablette | Gesamtansatz |
|---|---|---|
| Tramadol HCL | 100,0 mg | 2,0 g |
| Polyethylenoxid, NF, MG 7 000 000 (Polyox WSR 303, Dow Chemicals) | 221,0 mg | 4,42 g |
| Hydroxypropylmethylcellulose (Metholose 90 SH 100 000 cP von ShinEtsu) | 20,0 mg | 0,4 g |
| Butylhydroxytoluol (BHT) | 0,2 mg | 0,004 g |
| Gesamtgewicht | 341,2 mg | 6,824 g |

Die angegebene BHT-Menge wurde in Ethanol (96%) gelöst, so dass man eine 7,7%-ige (m/m) ethanolische Lösung erhielt. Diese wurde mit dem Polyethylenoxid gemischt und anschließend bei 40°C für 12 h getrocknet. Alle weiteren Komponenten wurden dieser getrockneten Mischung zugesetzt und in einem Freifallmischer für 15 min gemischt.

Die Herstellung der Tabletten erfolgte nach demselben Verfahren, wie es in Beispiel 1 angegeben. Es wurden runde Stempel (Durchmesser 10 mm) mit einem Wölbungsradius von 8 mm verwendet.

Die Bruchfestigkeit der Tabletten wurde nach der vorstehend beschriebenen Methode bestimmt. Bei einer Krafteinwirkung von 500 N trat kein Bruch auf. Die Tablette konnte weder mit einem Hammer noch mit Hilfe von Mörser und Pistill zerkleinert werden.

Die in vitro Freisetzung des Wirkstoffs aus der Darreichungsform wurde gemäß den Angaben in Beispiel 1 zur Bestimmung der Freisetzung durchgeführt.

| Zeit | Freigesetzte Wirkstoffmenge |
|---|---|
| 30 min | 17 % |
| 240 min | 59 % |
| 480 min | 86 % |
| 720 min | 98 % |

### Beispiel 8

| Komponenten | Pro Tablette | Gesamtansatz |
|---|---|---|
| Tramadol HCL | 100,0 mg | 2,0 g |
| Polyethylenoxid, NF, MG 7 000 000 (Polyox WSR 303, Dow Chemicals) | 221,0 | 4,42 g |
| Hydroxypropylmethylcellulose (Metholose 90 SH 100 000 cP von ShinEtsu) | 20,0 mg | 0,4 g |
| Gesamtgewicht | 341,0 mg | 6,82 g |

Die einzelnen Komponenten wurden in einem Freifallmischer für 15 Minuten gemischt. Die Herstellung der Tabletten erfolgte gemäß Beispiel 1 mit einem heißen Tablettierwerkzeug. Es wurden runde Stempel (Durchmesser 10 mm) mit einem Wölbungsradius von 8 mm verwendet.

Die Bruchfestigkeit der Tabletten wurde nach der angegebenen Methode bestimmt. Bei einer Krafteinwirkung von 500 N trat kein Bruch auf. Die Tablette konnte weder mit einem Hammer noch mit Hilfe von Mörser und Pistill zerkleinert werden.

Die in vitro Freisetzung des Wirkstoffs aus der Zubereitung wurde wie in Beispiel 1 angegeben bestimmt.

| Zeit | Freigesetzte Wirkstoffmenge |
|---|---|
| 30 min | 16 % |
| 240 min | 57 % |
| 480 min | 84 % |
| 720 min | 96% |

## Patentansprüche

1. Gegen Missbrauch gesicherte, ohne Extrusion thermogeformte Darreichungsform, welche neben einem oder mehreren Wirkstoffen mit Missbrauchspotential (A) sowie ggf. physiologisch verträglichen Hilfsstoffen (B) mindestens ein synthetisches oder natürliches Polymer (C) und ggf. mindestes ein Wachs (D) aufweist, wobei die Komponente(n) (C) und gegebenenfalls (D) in solchen Mengen vorliegt, dass die Darreichungsform eine Bruchfestigkeit von mindestens 500 N aufweist.

2. Darreichungsform gemäß Anspruch 1, **dadurch gekennzeichnet, dass** sie in Form einer Tablette vorliegt.

3. Darreichungsform gemäß Anspruch 1 oder 2, **dadurch gekennzeichnet, dass** sie als Polymer (C) wenigstens ein Polymer ausgewählt aus der Gruppe umfassend Polyethylenoxid, Polymethylenoxid, Polypropylenoxid, Polyethylen, Polypropylen, Polyvinylchlorid, Polycarbonat, Polystyrol, Polyacrylat, Copolymerisate und deren Mischungen, vorzugsweise Polyethylenoxid, enthält.

4. Darreichungsform gemäß einem der Ansprüche 1 bis 3, **dadurch gekennzeichnet, dass** das Polyethylenoxid (C) ein Molekulargewicht von mindestens 0,5 Mio. aufweist.

5. Darreichungsform gemäß Anspruch 4, **dadurch gekennzeichnet, dass** das Molekulargewicht des Polyethylenoxids (C) mindestens 1 Mio. beträgt.

6. Darreichungsform gemäß Anspruch 5, **dadurch gekennzeichnet, dass** das Molekulargewicht des Polyethylenoxids (C) 1-15 Mio. beträgt.

7. Darreichungsform gemäß einem der Ansprüche 1 bis 6, **dadurch gekennzeichnet, dass** sie als Wachs (D) wenigstens ein natürliches, halbsynthetisches oder synthetisches Wachs mit einem Erweichungspunkt von wenigstens 60°C enthält.

8. Darreichungsform gemäß Anspruch 7, **dadurch gekennzeichnet, dass** das Wachs (D) Camaubawachs oder Bienenwachs ist.

9. Darreichungsform gemäß einem der Ansprüche 1 bis 8, **dadurch gekennzeichnet, dass** der Wirkstoff (A) wenigstens ein Wirkstoff ausgewählt aus der Gruppe umfassend Opioide, Tranquillantien, Stimulatione, Barbiturate und weitere Betäubungsmittel ist.

10. Darreichungsform gemäß einem der Ansprüche 1 bis 9, **dadurch gekennzeichnet, dass** sie noch mindestens eine der nachfolgenden Komponenten a)-f) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wäßrigen Flüssigkeit aus der Darreichungsform gewonnenen Extrakt ein Gel bildet, welches vorzugsweise beim Einbringen in eine weitere Menge einer wäßrigen Flüssigkeit visuell unterscheidbar bleibt,
(c) wenigstens einen Antagonisten für den Wirkstoff bzw. die Wirkstoffe mit Missbrauchspotential,
(d) wenigstens ein Emetikum,
(e) wenigstens einen Farbstoff als aversives Mittel,
(f) wenigstens einen Bitterstoff.

11. Darreichungsform gemäß Anspruch 10, **dadurch gekennzeichnet, dass** die Komponente (b) wenigstens ein viskositätserhöhendes Mittel ausgewählt aus der Gruppe umfassend mikrokristalline Cellulose mit 11 Gew.-% Carboxymethylcellulose-Natrium (Avicel^{®} RC 591), Carboxymethylcellulose-Natrium (Blanose^{®} CMC-Na C300P^{®}, Frimulsion BLC-5^{®}, Tylose C300 P^{®}), Polyacrylsäure (Carbopol^{®} 980 NF, Carbopol^{®} 981), Johannisbrotkemmehl (Cesagum^{®} LA-200, Cesagum^{®} LID/150, Cesagum^{®} LN-1), Pektine aus Citrusfrüchten oder Äpfeln (Cesapectin^{®} HM Medium Rapid Set), Wachsmaisstärke (C*Gel 04201^{®)}, Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakernmehl (Polygum 43/1^{®}), Propylenglykoalginat (Protanal-Ester SD-LB^{®}), Apfelpektin, Natrium-Hyaluronat, Tragant, Taragummi (Vidogum SP 200^{®}), fermentiertes Polysaccharid- Welan Gum (K1A96) und Xanthan-Gummi (Xantural 180^{®}) ist.

12. Darreichungsform gemäß Anspruch 10 oder 11, **dadurch gekennzeichnet**, das die Komponente (c) wenigstens ein Opioid-Antagonist ausgewählt aus der Gruppe umfassend Naloxon, Naltrexon, Nalmefen, Nalid, Nalmexon, Nalorphin, Naluphin und eine entsprechende physiologisch verträgliche Verbindung, insbesondere eine Base, ein Salz oder Solvat ist.

13. Darreichungsform gemäß einem der Ansprüche 1 bis 12, **dadurch gekennzeichnet, dass** sie wenigstens einen Wirkstoff zumindest teilweise in retardierter Form enthält.

14. Darreichungsform gemäß Anspruch 13, **dadurch gekennzeichnet, dass** jeder der Wirkstoffe mit Missbrauchspotential (A) in einer Retardmatrix vorliegt.

15. Darreichungsform gemäß Anspruch 13, **dadurch gekennzeichnet, dass** die Komponente (C) und/oder die gegebenenfalls vorhandene Komponente (D) auch als Retardmatrixmaterial dient.

16. Verfahren zur Herstellung einer Darreichungsform gemäß einem der Ansprüche 1 bis 15, **dadurch gekennzeichnet, dass** man ohne Einsatz eines Extruders
die Komponenten (A), (B), (C) und die gegebenenfalls vorhandene Komponente (D) mischt sowie die ggf. vorhandenen Komponenten (a) bis (f) mitmischt oder, soweit notwendig, separat unter Zusatz der Komponente (C) und gegebenenfalls (D) mischt
und die resultierende Mischung oder die resultierenden Mischungen ggf. nach einer Granulierung zu der Darreichungsform unter vorangehender oder gleichzeitiger Wärmeeinwirkung durch Krafteinwirkung formt.

17. Verfahren gemäß Anspruch 16, **dadurch gekennzeichnet, dass** die Granulierung gemäß einer Schmelz- oder Feuchtgranulierung durchgeführt wird.

18. Darreichungsform nach einem der Ansprüche 1 bis 15 erhältlich nach Verfahren gemäß Anspruch 16 oder 17.

## Claims

1. An abuse-proofed dosage form thermoformed without extrusion, **characterised in that**, in addition to one or more active ingredients with abuse potential (A) optionally together with physiologically acceptable auxiliary substances (B), it contains at least one synthetic or natural polymer (C) and optionally at least one wax (D), wherein component(s) (C) and optionally (D) are present in quantities such that the dosage form exhibits a breaking strength of at least 500 N.

2. A dosage form according to claim 1, **characterised in that** it is in the form of a tablet.

3. A dosage form according to either one of claims 1 or 2, **characterised in that** it contains as polymer (C) at least one polymer selected from the group comprising polyethylene oxide, polymethylene oxide, polypropylene oxide, polyethylene, polypropylene, polyvinyl chloride, polycarbonate, polystyrene, polyacrylate, copolymers and the mixtures thereof, preferably polyethylene oxide.

4. A dosage form according to any one of claims 1 to 3, **characterised in that** the polyethylene oxide (C) has a molecular weight of at least 0.5 million.

5. A dosage according to claim 4, **characterised in that** the molecular weight of the polyethylene oxide (C) is at least 1 million.

6. A dosage form according to claim 5, **characterised in that** the molecular weight of the polyethylene oxide (C) is 1-15 million.

7. A dosage form according to any one of claims 1 to 6, **characterised in that** it contains as the wax (D) at least one natural, semi-synthetic or synthetic wax with a softening point of at least 60°C.

8. A dosage form according to claim 7, **characterised in that** the wax (D) is carnauba wax or beeswax.

9. A dosage form according to any one of claims 1 to 8, **characterised in that** the active ingredient (A) is at least one active ingredient selected from the group comprising opioids, tranquillisers, stimulants, barbiturates and further narcotics.

10. A dosage form according to any one of claims 1-9, **characterised in that** it additionally comprises at least one of the following components a)-f):
(a) at least one substance which irritates the nasal passages and/or pharynx,
(b) at least one viscosity-increasing agent, which, with the assistance of a necessary minimum quantity of an aqueous liquid, forms a gel with the extract obtained from the dosage form, which gel preferably remains visually distinguishable when introduced into a further quantity of an aqueous liquid,
(c) at least one antagonist for the active ingredient or active ingredients with abuse potential
(d) at least one emetic,
(e) at least one dye as an aversive agent,
(f) at least one bitter substance.

11. A dosage form according to claim 10, **characterised in that** the component (b) is at least one viscosity-increasing agent selected from the group comprising microcrystalline cellulose with 11 wt.% carboxymethylcellulose sodium (Avicel^{®} RC 591), carboxymethylcellulose sodium (Blanose^{®}, CMC-Na C300P^{®}, Frimulsion BLC-5^{®}, Tylose C300 P^{®}), polyacrylic acid (Carbopol^{®} 980 NF, Carbopol^{®} 981), locust bean flour (Cesagum^{®} LA-200, Cesagum^{®} LID/150, Cesagum^{®} LN-1), pectins from citrus fruit or apples (Cesapectin^{®} HM Medium Rapid Set), waxy maize starch (C*Gel 04201^{®}), sodium alginate (Frimulsion ALG (E401)^{®}), guar flour (Frimulsion BM^{®}, Polygum 26/1-75^{®}), iota carrageen (Frimulsion D021^{®}), karaya gum, gellan gum (Kelcogel F^{®}, Kelcogel LT100^{®}), galactomannan (Meyprogat 150 ^{®}), tara bean flour (Polygum 43/1^{®}), propylene glycol alginate (Protanal-Ester SD-LB^{®}), apple pectin, sodium hyaluronate, tragacanth, tara gum (Vidogum SP 200^{®}), fermented polysaccharide welan gum (K1A96), xanthan gum (Xantural 180^{®}).

12. A dosage form according to claim 10 or claim 11, **characterised in that** component (c) is at least one opioid antagonist selected from the group comprising naloxone, naltrexone, nalmefene, nalide, nalmexone, nalorphine, naluphine and a corresponding physiologically acceptable compound, in particular a base, a salt or solvate.

13. A dosage form according to any one of claims 1 to 12, **characterised in that** it contains at least one active ingredient at least partially in controlled release form.

14. A dosage form according to claim 13, **characterised in that** each of the active ingredients with abuse potential (A) is present in a controlled release matrix.

15. A dosage form according to claim 13, **characterised in that** component (C) and/or the optionally present component (D) also serve as a controlled release matrix material.

16. A process for the production of a dosage form according to any one of claims 1 to 15, **characterised in that**, without using an extruder,
components (A), (B), (C) and the optionally present component (D) are mixed and the optionally present components (a) to (f) are co-mixed or, if necessary, are separately mixed with addition of component (C) and optionally (D)
and the resultant mixture or mixtures, optionally after granulation, is/are shaped by application of force to yield the dosage form with preceding or simultaneous exposure to heat.

17. A process according to claim 16, **characterised in that** granulation is performed by melt granulation or wet granulation.

18. A dosage form according to any one of claims 1 to 15 obtainable by a process according to claim 16 or claim 17.

## Revendications

1. Forme d'administration protégée contre un usage abusif, thermoformée sans extrusion, qui présente, outre une ou plusieurs substances actives présentant un potentiel d'usage abusif (A) ainsi que le cas échéant des adjuvants physiologiquement compatibles (B), au moins un polymère synthétique ou naturel (C) et le cas échéant au moins une cire (D), le ou les composants (C) et le cas échéant (D) se trouvant en des quantités telles que la forme d'administration présente une résistance à la rupture d'au moins 500 N.

2. Forme d'administration selon la revendication 1, **caractérisée en ce qu'**elle se trouve sous forme d'un comprimé.

3. Forme d'administration selon la revendication 1 ou 2, **caractérisée en ce qu'**elle contient comme polymère (C) au moins un polymère choisi dans le groupe comprenant le poly(oxyde d'éthylène), le poly(oxyde de méthylène), le poly(oxyde de propylène), le polyéthylène, le polypropylène, le poly(chlorure de vinyle), le polycarbonate, le polystyrène, le polyacrylate, les copolymères et leurs mélanges, de préférence le poly(oxyde d'éthylène).

4. Forme d'administration selon l'une quelconque des revendications 1 à 3, **caractérisée en ce que** le poly(oxyde d'éthylène) (C) présente un poids moléculaire d'au moins 0,5 Mio.

5. Forme d'administration selon la revendication 4, **caractérisée en ce que** le poids moléculaire du poly(oxyde d'éthylène) (C) est d'au moins 1 Mio.

6. Forme d'administration selon la revendication 5, **caractérisée en ce que** le poids moléculaire du poly(oxyde d'éthylène) (C) est de 1-15 Mio.

7. Forme d'administration selon l'une quelconque des revendications 1 à 6, **caractérisée en ce qu'**elle contient comme cire (D) au moins une cire naturelle, semi-synthétique ou synthétique présentant un point de ramollissement d'au moins 60°C.

8. Forme d'administration selon la revendication 7, **caractérisée en ce que** la cire (D) est la cire de caroube ou la cire d'abeille.

9. Forme d'administration selon l'une quelconque des revendications 1 à 8, **caractérisée en ce que** la substance active (A) est au moins une substance active choisie dans le groupe comprenant les opiacés, les tranquillisants, les stimulants, les barbiturates et d'autres anesthésiants.

10. Forme d'administration selon l'une quelconque des revendications 1 à 9, **caractérisée en ce qu'**elle présente encore au moins un des composants suivants a)-f) :
(a) au moins une substance irritant le nez et/ou la gorge,
(b) au moins un agent augmentant la viscosité, qui forme dans un extrait obtenu de la forme d'administration à l'aide d'une quantité minimale nécessaire d'un liquide aqueux un gel qui reste de préférence visuellement distinguable lors de l'introduction dans une autre quantité d'un liquide aqueux,
(c) au moins un antagoniste pour la ou les substances actives avec un potentiel d'usage abusif,
(d) au moins un émétique,
(e) au moins un colorant comme agent d'aversion,
(f) au moins une substance amère.

11. Forme d'administration selon la revendication 10, **caractérisée en ce que** le composant (b) est au moins un agent augmentant la viscosité choisi dans le groupe comprenant la cellulose microcristalline comprenant 11% en poids de carboxyméthylcellulose sodique (Avicel^{®} RC 591), la carboxyméthylcellulose sodique (Blanose^{®}, CMC-Na C30OP^{®}, Frimulsion BLC-5^{®}, Tylose C300 P^{®}), le poly(acide acrylique) (Carbopol^{®} 980 NF, Carbopol^{®} 981), la farine de graines de caroube (Cesagum^{®} LA-200, Cesagum^{®} LID/150, Cesagum^{®} LN-1), la pectine provenant d'agrumes ou de pommes (Cesapectin^{®} HM Medium Rapid Set), l'amidon de maïs cireux (C*Gel 04201^{®}), l'alginate de sodium (Frimulsion ALG (E401)^{®}), la farine de graines de guar (Frimulsion BM^{®}, Polygum 26/1-75^{®}), le iota-carragheen (Frimulsion D021^{®}), la gomme de Karaya, la gomme de gellane (Kelcogel F^{®}, Kelcogel LT100^{®}), le galactomannane (Meyprogat 150^{®}), la farine de graines de tara (Polygum 43/1^{®}), le glycoalginate de propylène (Protanal-Ester SD-LB^{®}), la pectine de pomme, l'hyaluronate de sodium, la gomme adragante, la gomme de tara (Vidogum SP 200^{®}), la gomme de polysaccharide-welane fermentée (K1A96) et la gomme de xanthane (Xantural 180^{®}).

12. Forme d'administration selon la revendication 10 ou 11, **caractérisée en ce que** le composant (c) est au moins un antagoniste d'opiacés, choisi dans le groupe comprenant le Naloxon, le Naltrexon, le Nalmefen, le Nalid, le Nalmexon, la Nalorphine, la Naluphine et un composé correspondant physiologiquement acceptable, en particulier une base, un sel ou un solvate.

13. Forme d'administration selon l'une quelconque des revendications 1 à 12, **caractérisée en ce qu'**elle contient au moins une substance active qui se trouve au moins partiellement sous une forme de retard.

14. Forme d'administration selon la revendication 13, **caractérisée en ce que** chaque substance active avec un potentiel d'usage abusif (A) se trouve dans une matrice à effet de retard.

15. Forme d'administration selon la revendication 13, **caractérisée en ce que** le composant (C) et/ou le composant (D) le cas échéant présent sert également de matériau de matrice à effet de retard.

16. Procédé pour la préparation d'une forme d'administration selon l'une quelconque des revendications 1 à 15, **caractérisé en ce que**, sans l'addition d'une extrudeuse,
on mélange les composants (A), (B), (C) et le composant (D) le cas échéant présent en y incorporant les composants (a) à (f) le cas échéant présents ou, si nécessaire, on les mélange séparément avec addition du composant (C) et le cas échéant (D)
et on façonne le mélange obtenu ou les mélanges obtenus, le cas échéant après une granulation, en forme d'administration sous l'effet d'une force, sous l'effet préalable ou simultané de la chaleur.

17. Procédé selon la revendication 16, **caractérisé en ce que** la granulation est réalisée selon une granulation en masse fondue ou humide.

18. Forme d'administration selon l'une quelconque des revendications 1 à 15 pouvant être obtenue selon la revendication 16 ou 17.
